# EUROPEAN PATENT APPLICATION

(11) **EP 4 523 669 A1**
(43) Date of publication of application: **19.03.2025**
(21) Application number: 23849954.5
(22) Date of filing: 25.07.2023
(51) Int. Cl.: A61H 1/00

(54) **CONTROL DEVICE FOR VIBRATING BODY OF HAPTIC PRESENTATION DEVICE, AND HAPTIC PRESENTATION DEVICE**

(30) Priority: 01.08.2022 JP 2022122718
(71) Applicant: Murata Manufacturing Co., Ltd., Nagaokakyo-shi, Kyoto 617-8555 (JP)
(72) Inventor: NAKAMURA Norio, Nagaokakyo-shi, Kyoto 617-8555 (JP)
(74) Representative: Laufhütte, Dieter
(86) International application number: PCT/JP2023/027174
(87) International publication number: WO 2024/029399

(57) **Abstract**

A control unit (40) controls a vibrator (20) of a haptic device (10). The control unit (40) includes a CPU (41) as an execution unit and a storage (44). The storage (44) stores correlation data (CD) created by correlating multiple vibration modes and specifying information to each other. The vibration modes are used for presenting a tactile/force sense in accordance with a rehabilitation pattern. The specifying information is used for specifying a rehabilitation pattern. The CPU (41) executes obtaining processing, mode selection processing, and driving processing. In the obtaining processing, based on the correlation data (CD), the CPU (41) selects a vibration mode in accordance with specifying information obtained in the obtaining processing. In the mode selection processing, based on the correlation data (CD), the CPU (41) selects a vibration mode in accordance with specifying information obtained in the obtaining processing. In the driving processing, the CPU (41) drives the vibrator (20) by using the vibration mode selected in the mode selection processing.

## Description

### Technical Field

The present invention relates to a control unit for a vibrator of a haptic device and also to the haptic device.

### Background Art

A haptic device disclosed in Patent Document 1 includes a casing, a vibrator, and a control unit. The vibrator is disposed inside the casing. A user holds the casing with a hand, for example, to use the haptic device. The control unit performs control to set the vibration pattern of the vibrator to a specific pattern. The haptic device can thus present various tactile/force senses to the user holding the casing.

### Citation List

### Patent Document

Patent Document 1: Japanese Unexamined Patent Application Publication No. 2005-190465

### Summary of Invention

### Technical Problem

One of the situations where a user uses a haptic device such as that disclosed in Patent Document 1 may be that a user uses the haptic device as an assistive instrument to undergo rehabilitation to recover from a movement disorder. In this case, however, it is uncertain which type of tactile/force sense is to be presented to the user to enhance the effect of recovering from a movement disorder. Solution to Problem

To solve the above-described problem, an aspect of the present disclosure provides a control unit for a vibrator of a haptic device. The control unit includes a storage and an execution unit and controls the vibrator of the haptic device. The storage stores correlation data created by correlating multiple vibration modes of the vibrator and specifying information to each other. The vibration modes are used for presenting a tactile/force illusion sense in accordance with a rehabilitation pattern. The specifying information is used for specifying a rehabilitation pattern. The execution unit executes: obtaining processing for obtaining the specifying information; mode selection processing for selecting, based on the correlation data, a vibration mode of the multiple vibration modes in accordance with the specifying information obtained in the obtaining processing; and driving processing for driving the vibrator by using the vibration mode selected in the mode selection processing.

To solve the above-described problem, an aspect of the present disclosure provides a haptic device including a vibrator and a control unit. The control unit includes a storage and an execution unit and controls the vibrator. The storage stores correlation data created by correlating multiple vibration modes of the vibrator and specifying information to each other. The vibration modes are used for presenting a tactile/force sense in accordance with a rehabilitation pattern. The specifying information is used for specifying a rehabilitation pattern. The execution unit executes: obtaining processing for obtaining the specifying information; mode selection processing for selecting, based on the correlation data, a vibration mode of the multiple vibration modes in accordance with the specifying information obtained in the obtaining processing; and driving processing for driving the vibrator by using the vibration mode selected in the mode selection processing.

With the above-described configurations, in accordance with the specifying information, the vibrator of the haptic device can be vibrated in a vibration mode that presents a suitable tactile/force sense.

### Advantageous Effects of Invention

A suitable tactile/force sense can be presented in accordance with specifying information.

### Brief Description of Drawings

[Fig. 1] Fig. 1 is a schematic diagram illustrating a haptic device.
[Fig. 2] Fig. 2 is a schematic diagram illustrating correlation data.
[Fig. 3] Fig. 3 is a flowchart illustrating a series of operations executed in accordance with a control program.
[Fig. 4] Fig. 4 is a schematic diagram illustrating a setting system.
[Fig. 5] Fig. 5 is a flowchart illustrating a series of operations executed in accordance with a generation program. Description of Embodiments

### (Embodiment)

An embodiment of a control unit for a haptic (tactile/force sense presenting) device will be described below with reference to the drawings. The haptic device will first be explained below.

### <Haptic Device>

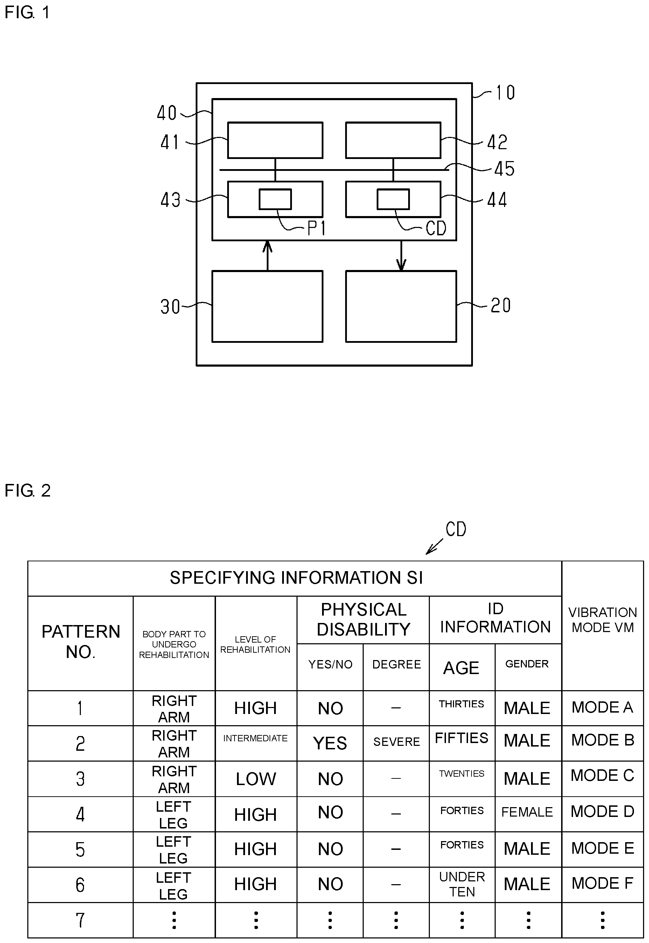

As illustrated in Fig. 1, a haptic device 10 includes a vibrator 20, an input/output unit 30, and a control unit 40.

The vibrator 20 is stored inside a casing of the haptic device 10, though such a state is not shown. The vibrator 20 includes voice coil motors, weights for the respective voice coil motors, and a cubic casing for storing the voice coil motors and the weights. A current flows through a coil of the voice coil motor to generate force, which vibrates the weight. In accordance with the vibration of the weight, the casing is vibrated. As a result of controlling a current to flow through the coil of the voice coil motor, the vibrator 20 is vibrated in a direction along the axis perpendicular to the surface of the casing. Specifically, the vibrator 20 is a vibrator such as that disclosed in Japanese Unexamined Patent Application Publication No. 2005-190465, for example.

The input/output unit 30 is a device used by a user to input specifying information SI for specifying a rehabilitation pattern. The input/output unit 30 is constituted by a touch screen display, for example. The input/output unit 30 can present information to a user in the form of an image. The input/output unit 30 is operated by a user. Specifying information SI is thus input into the input/output unit 30 by a user operation.

More specifically, the specifying information SI includes ID information for identifying a user, information indicating whether a user is physically disabled and the degree of physical disability, information on a body part to undergo rehabilitation, and information indicating the level of rehabilitation.

The control unit 40 controls the vibrator 20. The control unit 40 performs control to set the vibration pattern of the vibrator 20 to a specific vibration pattern corresponding to a tactile/force sense to be presented. The vibration pattern is a pattern of vibration represented by a nonlinear wave, for example. With this vibration pattern, the haptic device 10 presents a tactile sense or a force sense to a user. The tactile/force sense includes a tactile/force illusion sense. The tactile/force illusion sense includes a tactile illusion sense and a force illusion sense. The tactile illusion sense is the following type of sense of illusion. When vibrations of the vibrator 20 are given to a user, the user feels as if he/she were touching an uneven shape. The force illusion sense is the following type of sense of illusion. When vibrations of the vibrator 20 are given to a user, the user feels as if force were applied to the user.

The control unit 40 includes a CPU 41, which serves as an execution unit, a peripheral circuit 42, a ROM 43, a storage 44, and a bus 45. The bus 45 connects the CPU 41, peripheral device 42, ROM 43, and storage 44 so that they can communicate with each other. The peripheral circuit 42 includes various circuits, such as a circuit that generates a clock signal which determines the internal operation, a power supply circuit, and a reset circuit. In the ROM 43, various programs to be used by the CPU 41 to perform various control operations are stored. Among others, the ROM 43 stores a control program P1 to be used by the CPU 41 to perform control based on correlation data CD, which will be discussed later. The CPU 41 controls the vibrator 20 by executing various programs stored in the ROM 43.

The storage 44 stores correlation data CD. As shown in Fig. 2, the correlation data CD is data created by correlating multiple vibration modes VM and specifying information SI to each other. The vibration mode VM is used for presenting a tactile/force sense in accordance with a rehabilitation pattern. The specifying information SI is used for specifying a rehabilitation pattern. In the correlation data CD, one vibration mode VM is correlated to one pattern of specifying information SI.

The specifying information SI includes ID information for identifying a user, information indicating whether a user is physically disabled and the degree of physical disability, information on a body part to undergo rehabilitation, and information indicating the level of rehabilitation. In Fig. 2, for the specifying information SI, patterns NO. 1 through NO. 6 are shown by way of example, but specifying information SI may have any number of patterns. That is, only one pattern may be provided, or two or more patterns may be provided.

In the storage 44, data for implementing a vibration pattern of the vibrator 20 is stored. The tactile/force sense to be presented by the haptic device 10 is determined by this data stored in the storage 44. As a specific example, the storage 44 stores data for vibrating the vibrator 20 when the vibration mode VM is mode A. For instance, when the vibration mode VM is mode A, the CPU 41 vibrates the vibrator 20 based on this data so as to present a tactile/force sense corresponding to mode A to a user. In the example in Fig. 2, mode A through mode F are correlated to the respective patterns of specifying information SI. However, there may be an unregistered mode which is not correlated to any pattern of specifying information SI. In this case, when a new pattern of specifying information SI is created, this unregistered mode may be correlated to the new pattern. In another example, the same mode may be correlated to multiple patterns of specifying information SI.

### <Control Operation Based on Correlation Data>

As illustrated in Fig. 1, the CPU 41 executes the control program P1 stored in the ROM 43 so as to perform a series of operations for controlling the vibrator 20. The CPU 41 can thus execute obtaining processing for obtaining specifying information SI, mode selection processing for selecting a vibration mode VM based on correlation data CD, and driving processing for driving the vibrator 20.

When the haptic device 10 is powered ON, the CPU 41 executes the control program P1 stored in the ROM 43. The haptic device 10 is powered ON as a result of the input/output unit 30 being operated when the power of the haptic device 10 is OFF, for example. The control program P1 is a program for causing the CPU 41 to execute the obtaining processing, mode selection processing, and driving processing.

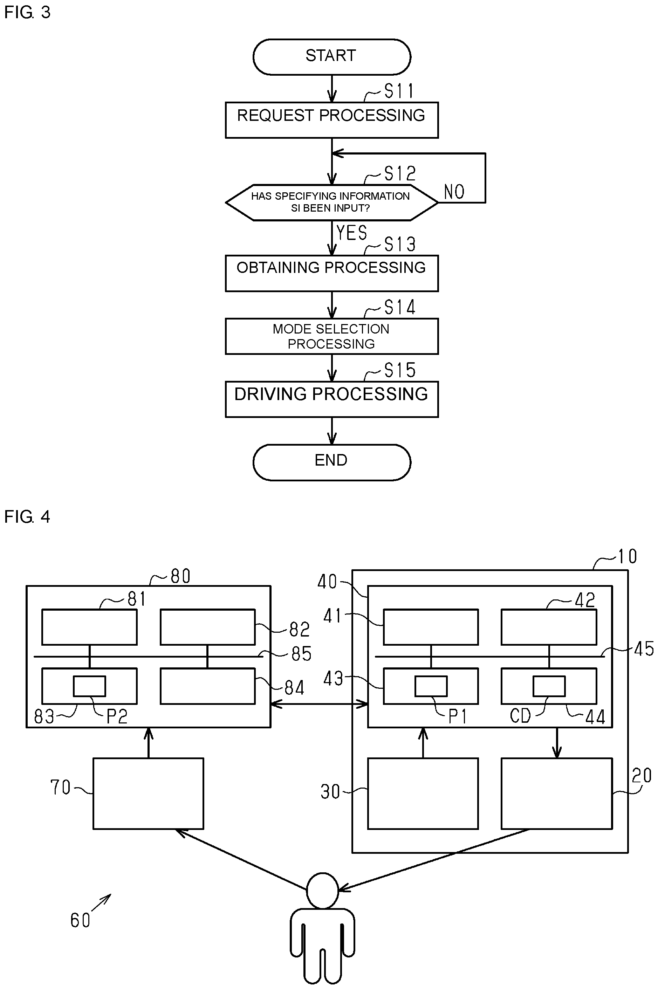

As illustrated in Fig. 3, after starting the control program P1, the CPU 41 first executes step S11. In step S11, the CPU 41 executes request processing. More specifically, in the request processing, the CPU 41 outputs image data of required items of specifying information SI and options that can be selected as each item of specifying information SI to the input/output unit 30 so that a user can select certain options. That is, the input/output unit 30 displays icons representing the items of specifying information SI and options for each item on the touch screen display.

More specifically, in the request processing, regarding the ID information for identifying a user, which is one item of the specifying information SI, the CPU 41 outputs ID information of registered users as options. In the embodiment, the age and the gender are linked to the ID information. In the request processing, regarding information indicating whether a user is physically disabled, which is one item of the specifying information SI, the CPU 41 outputs a "YES" option and a "NO" option to a YES/NO field of physical disability. If the user is physically disabled, the CPU 41 outputs a field of information indicating the degree of physical disability and options of three degrees, such as "severe", "intermediate", and "mild", as options of this information. In the request processing, regarding information on a body part to undergo rehabilitation, which is one item of the specifying information SI, the CPU 41 outputs multiple body parts, such as the right arm and the left leg, as options. Regarding the level of rehabilitation, the CPU 41 outputs, for example, options of three levels, such as "high", "intermediate", and "low". The above-described options may be displayed at the same time on one screen, or every time one option is selected, the next options may be displayed. The CPU 41 then proceeds to step S12.

In step S12, the CPU 41 determines whether the user has input information using the input/output unit 30. More specifically, the CPU 41 determines whether the user has selected options for all the items of specifying information SI whose options have been output in the request processing. If the user has selected options for all the items of specifying information SI, the CPU 41 determines that the user has input information using the input/output unit 30. If the user has not selected options for some of the items of specifying information SI, the CPU 41 determines that the user has not yet input information using the input/output unit 30.

If the user has not yet input information using the input/output unit 30 (S12: NO), the CPU 41 repeats step S12. If the user has input information using the input/output unit 30 (S12: YES), the CPU 41 proceeds to step S13.

In step S13, the CPU 41 executes obtaining processing. In the obtaining processing, the CPU 41 obtains specifying information SI. More specifically, the CPU 41 obtains, as specifying information SI of the user, the options selected as the individual items of specifying information SI input into the input/output unit 30. The CPU 41 then proceeds to step S14.

In step S14, the CPU 41 executes mode selection processing. In the mode selection processing, based on the correlation data CD, the CPU 41 selects a vibration mode VM corresponding to the specifying information SI of the user obtained in the obtaining processing. In the correlation data CD, one vibration mode VM is correlated to one set of specifying information SI. Based on the correlation data CD, the CPU 41 selects the vibration mode VM corresponding to the specifying information SI of the user obtained in the obtaining processing. The CPU 41 then proceeds to step S15.

In step S15, the CPU 41 executes driving processing. In the driving processing, the CPU 41 drives the vibrator 20 by using the vibration mode VM selected in the mode selection processing. While the haptic device 10 is being driven in this manner in the vibration mode VM corresponding to the specifying information SI, the user undergoes rehabilitation by using the haptic device 10 as an assistive instrument. That is, the user performs physical exercises related to the rehabilitation while receiving the presentation of a tactile/force sense from the haptic device 10. After executing the driving processing for a certain time, the CPU 41 completes a series of operations.

### <Generation Method for Correlation Data>

A generation method for the correlation data CD will be described below. A setting system 60 that generates the correlation data CD will first be discussed.

As illustrated in Fig. 4, the setting system 60 includes the above-described haptic device 10, a measuring device 70, and a setting device 80. The devices of the setting system 60 are connected to each other so as to communicate with each other.

The measuring device 70 is a device that measures an activation parameter indicating the activation state of a user having received a tactile/force sense from the haptic device 10. For example, the measuring device 70 measures the activation state of the brain of a user. More specifically, the measuring device 70 is a brain measuring instrument using near-infrared spectroscopy.

The measuring device 70 measures activation parameters of individual portions of the brain. For example, the measuring device 70 measures the blood flow rate of an individual portion of the brain as the activation parameter. As the blood flow rate is higher, the degree of the brain activation is higher. The measuring device 70 measures the activation parameters of portions of the primary motor cortex of the brain corresponding to individual body parts. The measuring device 70 then sends the activation parameters of the individual portions of the brain to the setting device 80.

The setting device 80 is a device for generating the correlation data CD. The setting device 80 controls the vibrator 20 of the haptic device 10 via the control unit 40. The setting device 80 includes a CPU 81, a peripheral circuit 82, a ROM 83, a storage 84, and a bus 85. The bus 85 connects the CPU 81, peripheral device 82, ROM 83, and storage 84 so that they can communicate with each other. The peripheral circuit 82 includes various circuits, such as a circuit that generates a clock signal which determines the internal operation, a power supply circuit, and a reset circuit. In the ROM 83, various programs to be used by the CPU 81 to perform various control operations are stored. Among others, a generation program P2 for generating the correlation data CD is stored in the ROM 83. The CPU 81 generates the correlation data CD by executing the generation program P2 stored in the ROM 83.

The CPU 81 executes the generation program P2 stored in the ROM 83 so as to perform a series of operations for generating the correlation data CD. The CPU 81 can execute test driving processing, parameter obtaining processing, and a mode determining processing, all of which will be discussed later. When the haptic device 10 is powered ON in a state in which the haptic device 10, the measuring device 70, and the setting device 80 are connected to each other, the CPU 81 executes the generation program P2 stored in the ROM 83. That is, the generation program P2 is a program for causing the CPU 81 to execute the test driving processing, parameter obtaining processing, and a mode determining processing.

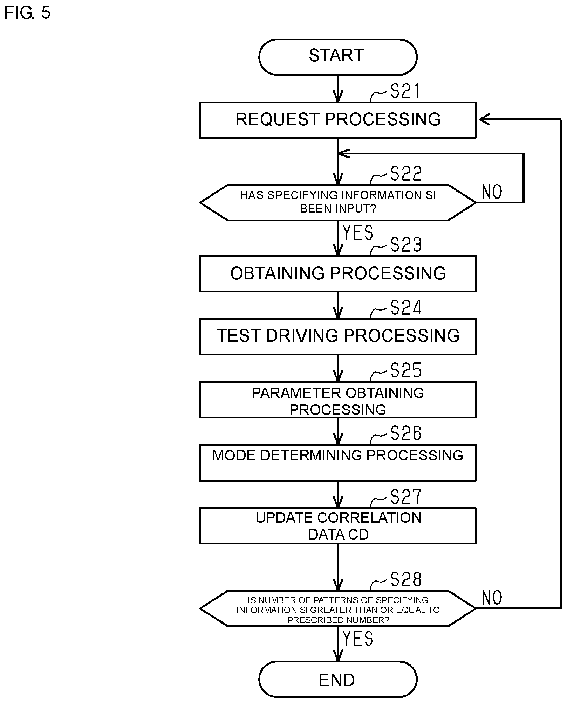

As illustrated in Fig. 5, after starting the generation program P2, the CPU 81 first executes steps S21 through S23. Steps S21 through S23 are similar to steps S11 through S13 of the above-described control program P1. After step S23, the CPU 81 proceeds to step S24.

In step S24, the CPU 81 starts test driving processing. In the test driving processing, the CPU 81 drives the vibrator 20 in multiple vibration modes VM in a predetermined order for a predetermined period. In the test driving processing, the CPU 81 starts measuring the activation state of the brain of a user by using the measuring device 70. During the execution of the test driving processing, the user preferably undergoes rehabilitation using the haptic device 10 as an assistive instrument. The CPU 81 then proceeds to step S25.

In step S25, the CPU 81 executes parameter obtaining processing. In the parameter obtaining processing, the CPU 81 obtains the activation parameters which were measured by the measuring device 70 when the vibrator 20 was vibrated in the individual vibration modes VM. After the parameter obtaining processing has been completed, the CPU 81 proceeds to step S26.

In step S26, the CPU 81 executes mode determining processing. In the mode determining processing, the CPU 81 determines a specific mode, which is a specific vibration mode VM, from among multiple vibration modes VM, based on the activation parameters measured for the individual vibration modes VM in the parameter obtaining processing.

This will be explained more specifically. First, the CPU 81 determines the largest value among the activation parameters of the individual brain parts that are obtained while the vibrator 20 was vibrated in the first vibration mode VM. The CPU 81 then sets the part of the brain having the largest value to be the most activated part. Then, the CPU 81 determines the most activated part in the second vibration mode VM in a manner similar to the first vibration mode VM. The CPU 81 repeats this operation to calculate the most activated part in each vibration mode VM.

Then, the CPU 81 refers to the specifying information SI obtained in the obtaining processing in step S23 to see information on the body part to undergo rehabilitation and checks this information against the most activated parts in the individual vibration modes VM. The CPU 81 then gives a score to each vibration mode VM based on the checking results.

More specifically, a body part of a user to undergo rehabilitation, such as the right arm and the left leg, corresponds to a certain brain part which functions when the user moves this body part. If the most activated part of a certain vibration mode VM matches the brain part corresponding to the body part which undergoes rehabilitation, the CPU 81 adds ten points to the score of this vibration mode VM.

If there are vibration modes VM whose most activated parts match the brain part corresponding to the body part which undergoes rehabilitation, the CPU 81 adds five points to the score of the vibration mode VM having the largest activation parameter of the most activated part.

Then, the CPU 81 refers to information on the level of rehabilitation in the specifying information SI obtained in the obtaining processing in step S23, and checks this information against the activation parameters of the most activated parts of the individual vibration modes VM. If the activation parameter of the most activated part of a certain vibration mode VM is contained within a preset range including a value necessary for this level of rehabilitation, the CPU 81 adds four points to the score of this vibration mode VM.

Then, the CPU 81 refers to information indicating whether the user is physically disabled and the degree of physical disability in the specifying information SI obtained in the obtaining processing in step S23, and checks this information, the body part to undergo rehabilitation, and the individual vibration modes VM against each other. The CPU 81 then resets the score of a vibration mode VM which is preset in accordance with whether the user is physically disabled and the degree of physical disability to 0 points, regardless of whether certain points have been added to the score. Such a vibration mode VM is an unsuitable vibration mode VM which is determined based on whether the user is physically disabled and the degree of physical disability. For example, if the user is physically disabled and the degree of physical disability is severe, the score of the vibration mode VM set for the rehabilitation for an able-bodied person is reset to 0 points. Such a vibration mode VM is determined based on testing, simulations, and experiences of healthcare professionals, such as medical doctors.

Then, the CPU 81 refers to information on the ID information for identifying the user in the specifying information SI obtained in the obtaining processing in step S23, and checks this information against the individual vibration modes VM. The CPU 81 then resets the score of a vibration mode VM which is preset in accordance with the user ID information to 0 points. Such a vibration mode VM does not have enough effects for a user of a certain gender or a certain age. For example, if the age of a user is under ten or 80 or over, the score of a vibration mode VM having a certain strength of a tactile/force sense is reset to 0 points. Such a vibration mode VM is determined based on testing, simulations, and experiences of healthcare professionals, such as medical doctors. Then, among the plural vibration modes VM, the CPU 81 sets the vibration mode VM having the highest score to be the specific mode. The CPU 81 then proceeds to step S27.

In step S27, the CPU 81 correlates the specifying information SI obtained in step S23 to the vibration mode VM determined in step S26 and stores the resulting correlation data CD in the storage 44. If the correlation data CD representing the same pattern of specifying information SI is already stored, the CPU 81 updates the correlation data CD. The CPU 81 then proceeds to step S28.

In step S28, the CPU 81 determines whether the number of patterns of specifying information SI of the correlation data CD is greater than or equal to a prescribed number, which represents the number of sets of specifying information SI. If the number of patterns of specifying information SI of the correlation data CD is smaller than the prescribed number (S28: NO), the CPU 81 repeats steps S21 through S27 for another user. If the number of patterns of specifying information SI of the correlation data CD is greater than or equal to the prescribed number (S28: YES), the CPU 81 completes a set of operations.

### <Operation of Embodiment>

According to the embodiment, in the correlation data CD, the vibration mode VM is correlated to specifying information SI so that a tactile/force sense suitable for a user to undergo rehabilitation can be presented to the user. The user inputs his/her own specifying information SI when using the haptic device 10. It is assumed that, for example, as user ID information, the user has input specifying information SI indicating that the body part to undergo rehabilitation is the right arm, the level of rehabilitation is high, the user is not physically disabled, and the user is a male in his thirties. In this case, the control unit 40 obtains this specifying information SI. The CPU 41 selects mode A as the vibration mode VM correlated to this specifying information SI, based on the correlation data CD. It is assumed that, for example, as user ID information, a user has input specifying information SI indicating that the body part to undergo rehabilitation is the right arm, the level of rehabilitation is intermediate, the user is physically disabled and the degree of disability is severe, and the user is a male in his fifties. In this case, the control unit 40 obtains this specifying information SI. The CPU 41 selects mode B as the vibration mode VM correlated to this specifying information SI, based on the correlation data CD. In this manner, the haptic device 10 selects the vibration mode VM in accordance with the specifying information SI of a user.

### <Advantages of Embodiment>

(1) In the above-described embodiment, in the mode selection processing, the haptic device 10 selects the vibration mode VM which reflects the specifying information SI obtained in obtaining processing, based on the correlation data CD. Hence, in accordance with the specifying information SI of a user, the haptic device 10 can drive the vibrator 20 by using the vibration mode VM which presents a suitable tactile/force sense to the user. That is, the user inputs his/her specifying information SI and can undergo rehabilitation while receiving the presentation of a suitable tactile/force sense among the tactile/force senses that the haptic device 10 can present.
(2) In the above-described embodiment, the specifying information SI includes information indicating the level of rehabilitation. The haptic device 10 can thus present a suitable tactile/force sense corresponding to the required level of rehabilitation in accordance with the symptom of a patient.
(3) In the above-described embodiment, the specifying information SI includes information indicating a body part to undergo rehabilitation. The haptic device 10 can thus present a suitable tactile/force sense in accordance with the body part to undergo rehabilitation.
(4) In the above-described embodiment, the specifying information SI includes information indicating whether a user is physically disabled and the degree of physical disability. The haptic device 10 can thus avoid selecting a vibration mode VM that may be unsuitable for the degree of the physical disability of a user.
(5) In the above-described embodiment, the specifying information SI includes ID information of a user. Hence, based on information linked with the user ID information, such as the age of the user, the haptic device 10 can avoid selecting a vibration mode VM that may be unsuitable for the age of the user.
(6) According to the generation method for correlation data CD in the above-described embodiment, the correlation data CD is generated based on the activation state of the body of a user, more specifically, the activation states of individual parts of the brain of the user, at the time of driving the vibrator 20 in multiple vibration modes **VM.** In the mode determining processing, therefore, the CPU 81 can determine the vibration mode VM that is highly likely to activate the brain when the user undergoes rehabilitation.
(7) When a tactile/force sense is presented to a user, even if some body parts of the user, such as the arms and the legs, are not physically moved, portions of the primary motor cortex of the brain corresponding to such body parts may be activated. According to the generation method for correlation data CD in the above-described embodiment, the measuring device 70 measures activation parameters of individual parts of the brain of a user. This makes it possible to obtain activation parameters of brain parts even when the corresponding body parts are not physically moved.

### (Other Embodiments)

The above-described embodiment may be modified in the following manner and be carried out. The embodiment and the following modified examples may be combined with each other and be carried out as long as the resulting configurations do not become technically inconsistent.

In the above-described embodiment, the configuration of the vibrator 20 is not limited to that of the embodiment. For example, the vibrator 20 may be a vibrator using vibrations of a motor or a vibrator including a piezoelectric element.

Plural vibrators 20 may be provided. In this case, the vibration mode VM may include information indicating whether each of the vibrators 20 is vibrated, the strength of the vibration, and the vibration order of the vibrators 20. This can enhance the variety of patterns of vibration modes VM.

In the above-described embodiment, the control unit 40 is not limited to a device that includes a CPU and a ROM and executes software processing. For example, some of the software operations in the above-described embodiment may be executed by a hardware circuit (an ASIC, for example) dedicated to hardware processing. That is, the control unit 40 may have any one of the following configurations (a) through (c): (a) the control unit 40 includes a processor that executes all the above-described processing operations in accordance with a program and a program storage unit, such as a ROM; (b) the control unit 40 includes a processor that executes some of the above-described processing operations in accordance with a program, a program storage unit, and a dedicated hardware circuit that executes the remaining processing operations; and (c) the control unit 40 includes a dedicated hardware circuit that executes all the above-described processing operations. In the above-described configurations, multiple software execution units each including a processor and a program storage unit and multiple dedicated hardware circuits may be provided. Likewise, the configuration of the setting device 80 may be modified similarly to the control unit 40.

The specifying information SI may be information merely for specifying a rehabilitation pattern. For example, information indicating the level of rehabilitation may be omitted from the specifying information SI. In another example, information indicating a body part to undergo rehabilitation may be omitted from the specifying information SI. In another example, information indicating whether a user is physically disabled and the degree of physical disability may be omitted from the specifying information SI. In another example, ID information for identifying a user may be omitted from the specifying information SI. The specifying information SI may include information other than those indicated in the embodiment. For example, the specifying information SI may include information indicating the state of the physical disability of a user. An example of such information is that indicating whether a user has a severe physical disability.

The structure of the correlation data CD may have a format other than a table format, such as that shown in Fig. **2****.** For example, the structure of the correlation data CD may be such that multiple tables are provided for each body part to undergo rehabilitation. In another example, the structure of the correlation data CD may be such that one vibration mode VM may be correlated to one item of specifying information SI.

In the correlation data CD, multiple vibration modes VM may be correlated to one set of specifying information SI. In this case, the CPU 41 selects multiple vibration modes VM in the vibration mode selection processing and then sequentially drives the vibrator 20 in the selected vibration modes VM in the driving processing. Alternatively, in the driving processing, the CPU 41 may generate a specific vibration pattern by combining multiple vibration modes VM.

The correlation data CD is not limited to data generated by the generation method in the embodiment. The control program P1 may be executed using correlation data CD generated by another generation method.

The vibration mode VM may be a mode that presents only a tactile sense or a mode that presents only a force sense. In the request processing, instead of outputting options, a user may be instructed to input data in text format. The content of request processing may be suitably changed in accordance with the input/output unit 30.

In step S12, if an input completion button is displayed on the input/output unit 30, when this input completion button is pressed, the CPU 41 may assume that a user has finished inputting information.

The measuring device 70 is not limited to a brain measuring instrument using near-infrared spectroscopy. For example, the measuring device 70 may be a measuring instrument using functional magnetic resonance imaging (MRI). In another example, the measuring device 70 may be a measuring instrument using electroencephalography (EEG).

The measuring device 70 is not necessarily a device that measures the activation state of the brain of a user. For example, the measuring device 70 may be a device that measures the activation state of a specific body part other than the brain. As a specific example, if the body part of a user to undergo rehabilitation is the right leg, the measuring device 70 may be a device that measures the activation state of the right leg.

In the generation method for the correlation data CD, scores may be given to vibration modes VM differently from the example discussed in the embodiment. For instance, if a certain vibration mode VM is highly evaluated by healthcare professionals as a vibration mode that produces a more effective tactile/force sense, a higher score may be given to this vibration mode VM.

The setting device 80 may control the vibrator 20 without the intervention of the control unit 40. For example, if the storage 84 of the setting device 80 stores data indicating multiple vibration modes VM, the CPU 81 may drive the vibrator 20 by using this data in the test driving processing.

The technical concepts that can be derived from the above-described embodiment and modified examples will be described below.

### <Appendix 1>

A control unit for a vibrator of a haptic device, comprising:
a storage; and
an execution unit, wherein
the control unit controls the vibrator of the haptic device,
the storage stores correlation data, the correlation data being data created by correlating a plurality of vibration modes of the vibrator and specifying information to each other, the plurality of vibration modes being used for presenting a tactile/force sense in accordance with a rehabilitation pattern, the specifying information being used for specifying a rehabilitation pattern, and
the execution unit executes
   obtaining processing for obtaining the specifying information,
   mode selection processing for selecting, based on the correlation data, a vibration mode of the plurality of the vibration modes in accordance with the specifying information obtained in the obtaining processing, and
   driving processing for driving the vibrator by using the vibration mode selected in the mode selection processing.

### <Appendix 2>

The control unit according to <Appendix 1>, wherein the specifying information includes information indicating a level of rehabilitation.

### <Appendix 3>

The control unit according to <Appendix 1> or <Appendix 2>, wherein the specifying information includes information indicating a body part to undergo rehabilitation.

### <Appendix 4>

The control unit according to one of <Appendix 1> to <Appendix 3>, wherein the specifying information includes information indicating whether a user is physically disabled and a degree of physical disability.

### <Appendix 5>

The control unit according to one of <Appendix 1> to <Appendix 4>, wherein the specifying information includes information indicating a state of physical disability of a user.

### <Appendix 6>

The control unit according to one of <Appendix 1> to <Appendix 5>, wherein the specifying information includes ID information for identifying a user.

### <Appendix 7>

The control unit according to one of <Appendix 1> to <Appendix 6>, wherein:
the haptic device includes a plurality of the vibrators; and
the vibration modes include information regarding whether each of the plurality of the vibrators is vibrated, strength of the vibration, and a vibration order of the vibrators.

### <Appendix 8>

A method for generating correlation data, the correlation data being data created by correlating a plurality of vibration modes and specifying information to each other, the plurality of vibration modes being used for presenting a tactile/force sense in accordance with a rehabilitation pattern, the specifying information being used for specifying a rehabilitation pattern, the method comprising:
obtaining processing for obtaining the specifying information;
test driving processing for driving a vibrator in a plurality of the vibration modes;
parameter obtaining processing for obtaining an activation parameter indicating an activation state of a user who has received presentation of a tactile/force sense when the vibrator is driven in each of the vibration modes in the test driving processing;
mode determining processing for determining a specific mode, which is a specific vibration mode selected from a plurality of the vibration modes, based on the activation parameter obtained for each of the vibration modes in the parameter obtaining processing; and
updating processing for updating the correlation data by correlating the specifying information obtained in the obtaining processing and the specific mode determined in the mode determining processing to each other.

### <Appendix 9>

The method according to <Appendix 8>, wherein the activation parameter obtained in the parameter obtaining processing from the user who has received presentation of a tactile/force sense when the vibrator is driven in each of the vibration modes in the test driving processing is an activation parameter indicating an activation state of a portion of a primary motor cortex of a brain of the user corresponding to a certain body part of the user.

### <Appendix 10>

The method according to <Appendix 9>, wherein:
the specifying information includes information indicating a body part to undergo rehabilitation; and
in the mode determining processing, among the activation parameters obtained in the parameter obtaining processing, the specific mode is determined based on the activation parameter of a portion of the brain corresponding to the body part to undergo rehabilitation.

### Reference Signs List

- 10: haptic device
- 20: vibrator
- 30: input/output unit
- 40: control unit
- 41: CPU
- 42: peripheral circuit
- 43: ROM
- 44: storage
- 45: bus
- 60: setting system
- 70: measuring device
- 80: setting device
- CD: correlation data
- SI: specifying information
- VM: vibration mode

## Claims

1. A control unit for a vibrator of a haptic device, comprising:
a storage; and
an execution unit, wherein
the control unit controls the vibrator of the haptic device,
the storage stores correlation data, the correlation data being data created by correlating a plurality of vibration modes of the vibrator and specifying information to each other, the plurality of vibration modes being used for presenting a tactile/force sense in accordance with a rehabilitation pattern, the specifying information being used for specifying a rehabilitation pattern, and
the execution unit executes
obtaining processing for obtaining the specifying information,
mode selection processing for selecting, based on the correlation data, a vibration mode of the plurality of the vibration modes in accordance with the specifying information obtained in the obtaining processing, and
driving processing for driving the vibrator by using the vibration mode selected in the mode selection processing.

2. The control unit according to Claim 1, wherein the specifying information includes information indicating a level of rehabilitation.

3. The control unit according to Claim 1 or 2, wherein the specifying information includes information indicating a body part to undergo rehabilitation.

4. The control unit according to one of Claims 1 to 3, wherein the specifying information includes information indicating whether a user is physically disabled and a degree of physical disability.

5. The control unit according to one of Claims 1 to 4, wherein the specifying information includes information indicating a state of physical disability of a user.

6. The control unit according to one of Claims 1 to 5, wherein the specifying information includes ID information for identifying a user.

7. The control unit according to one of Claims 1 to 6, wherein:
the haptic device includes a plurality of the vibrators; and
the vibration modes include information regarding whether each of the plurality of the vibrators is vibrated, strength of the vibration, and a vibration order of the vibrators.

8. A haptic device comprising:
a vibrator; and
a control unit that includes a storage and an execution unit and controls the vibrator, wherein
the storage stores correlation data, the correlation data being data created by correlating a plurality of vibration modes of the vibrator and specifying information to each other, the plurality of vibration modes being used for presenting a tactile/force sense in accordance with a rehabilitation pattern, the specifying information being used for specifying a rehabilitation pattern, and
the execution unit executes
obtaining processing for obtaining the specifying information,
mode selection processing for selecting, based on the correlation data, a vibration mode of the plurality of the vibration modes in accordance with the specifying information obtained in the obtaining processing, and
driving processing for driving the vibrator by using the vibration mode selected in the mode selection processing.
